# EUROPEAN PATENT SPECIFICATION

(11) **EP 0 532 058 B1**
(45) Date of publication and mention of the grant of the patent: **14.12.1994**
(21) Application number: 92117140.1
(22) Date of filing: 07.07.1988
(51) Int. Cl.: C07D 213/82, C07D 213/89, A01N 47/36

(54) **Process for preparing halogenosulfonyl-substituted pyridine compounds**
Verfahren zur Herstellung von Halogensulfonyl-substituierten Pyridinen
Procédé pour la préparation de pyridines halogénosulfonyl substituées

(30) Priority: 10.07.1987 JP 172452/87; 24.02.1988 JP 41269/88; 26.03.1988 JP 72771/88
(43) Date of publication of application: 17.03.1993
(62) Divisional of application: 88306238.2
(73) Proprietor: ISHIHARA SANGYO KAISHA, LTD., Nishi-ku Osaka (JP)
(72) Inventor: Haga, Takahiro, c/o Ishihara Sangyo, Kusatsu-shi, Shiga-ken (JP); Isogai, Tatsuo, c/o Ishihara Sangyo, Kusatsu-shi, Shiga-ken (JP); Tsujii, Yasuhiro, c/o Ishihara Sangyo, Kusatsu-shi, Shiga-ken (JP); Murai, Shigeo, c/o Ishihara Sangyo, Kusatsu-shi, Shiga-ken (JP); Jyonishi, Hisayoshi, c/o Ishihara Sangyo, Kusatsu-shi, Shiga-ken (JP); Sasaki, Hiroshi, c/o Ishihara Sangyo, Kusatsu-shi, Shiga-ken (JP); Kimura, Tokiya, c/o Ishihara Sangyo, Kusatsu-shi, Shiga-ken (JP)
(74) Representative: Pearce, Anthony Richmond

(56) References cited:
- EP-A- 0 142 811
- EP-A- 0 232 067
- EP-A- 0 237 292
- DE-A- 3 342 538
- DE-C- 550 685
- US-A- 4 435 206
- JOURNAL OF THE AMERICAN CHEMICAL SOCIETY. vol. 64, 1942, GASTON, PA US pages 1695 - 1698 W.T. CALDWELL ET AL. 'Substituted 2-sulfonamido-5-aminopyridines'
- JOURNAL OF THE AMERICAN CHEMICAL SOCIETY. vol. 72, 1950, GASTON, PA US pages 4890 - 4892 R.O. ROBLIN ET AL. 'The preparation of heterocyclic sulfonamides'
- SAUL PATAI 'The chemistry of the thiol group part 2, pages 791-795' 1974 , JOHN WILEY & SONS, LONDON
- JOURNAL OF THE AMERICAN CHEMICAL SOCIETY. vol. 60, 1938, GASTON, PA US pages 1486 - 1489 I.B. DOUGLASS 'The interaction of chlorine with different types of organic sulfur compounds'

## Description

The present invention relates to a process for preparing halogenosulfonyl-substituted pyridine compounds.

J. Am. Chem. Soc. 64, **1942**, 1965-1968 discloses a process for preparing 5-acetamino-2-pyridinesufonyl chloride by dissolving 2-thiol-5-acetaminopyridine in cold concentrated hydrochloric acid, adding ice water and then bubbling chlorine into the solution whilst maintaining the temperature below 10°C.

J. Am. Chem. Soc. 72,**1950**, 4890-4892 discloses a process for preparing heterocyclic sulfonyl chlorides by low temperature oxidative chlorination of the corresponding thiol.

According to the present invention, there is provided a process for producing a compound of the general formula (A): 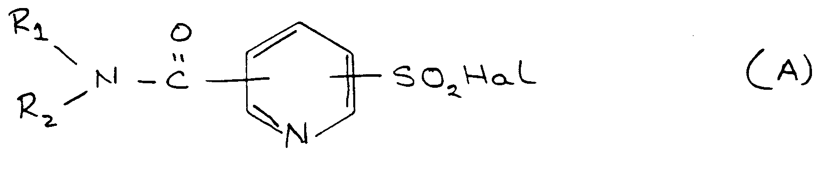
wherein each of R₁ and R₂ is C₁-C₆ alkyl group and Hal is chlorine or bromine;
said process comprising oxidising and halogenating a compound of the general formula (B): 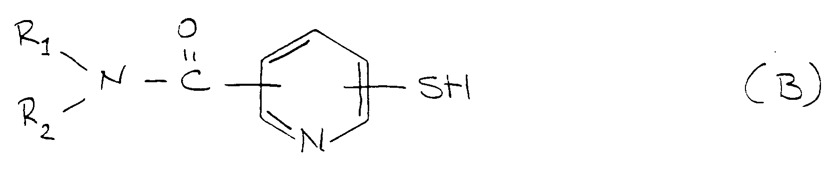
wherein R₁ and R₂ are as defined above, or a salt thereof.

The compound of the general formula (B) has an aminocarbonyl group on the pyridine ring, said aminocarbonyl group being substituted optionally by one or two alkyl groups. Certain substituted pyridine compounds are disclosed in the respective specifications of U.S.P. 3,759,932; U.S.P. 4,435,206; U.S.P. 4, 518,776 and U.S.P. 4,521,597. However, the above specifications fail to disclose the aforementioned mercapto-substituted pyridine compound on the pyridine ring.

The C₁-C₆ alkyl group in R₁ and R₂ of the mercapto-substituted pyridine compound of the general formula (B) includes a methyl group, ethyl group, propyl group and butyl group.

The salt of the mercapto-substituted pyridine compound includes acid addition salts, alkali metal salts and the like, and specifically includes salts of the mercapto-substituted pyridine compounds of the general formula (B) with an alkali metal such as lithium, sodium, potassium or the like.

Among the mercapto-substituted pyridine compounds and salts thereof, the compounds having the general formula (B'): 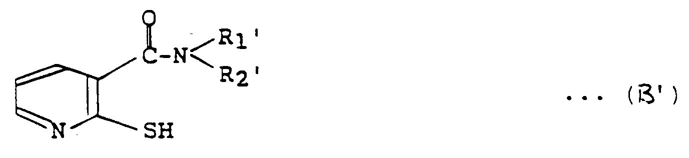
wherein each of R₁, and R₂, is a C₁-C₆ alkyl group, and further such a combination that R₁, is a methyl group and R₂, is a methyl group in the general formula (B'), is more preferred.

The mercapto-substituted pyridine compound of the general formula (B) may be prepared, as shown by the following equation: 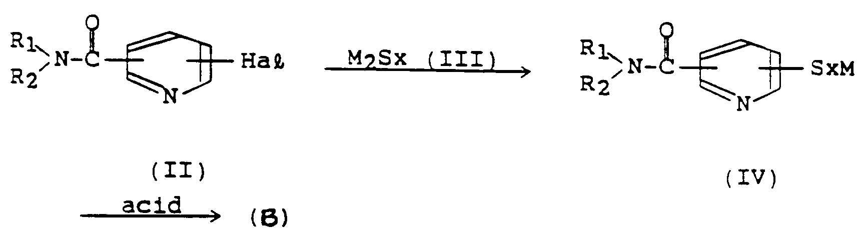
where M is an alakli metal, x is 2 to 8, Hal is a halogen atom, and R₁ and R₂ are defined as above, by a synthetic reaction of pyridinepolysulfide in which a halogeno-substituted pyridine compound having the general formula (II) is reacted with a polysulfide having the general formula (III) followed by an acid treatment.

### (Preparation of the halogeno-substituted pyridine compound)

The halogeno-substituted pyridine compound may be easily prepared, for example, by reacting thionyl chloride with a halogenopicolinic acid or halogeno-nicotinic acid or halogenoisonicotinic acid followed by reacting with an amine in a solvent such as methylene chloride. Among the halogeno-substituted pyridine compounds, such ones that the halogen atom located at the 2, 4 or 6 position of the pyridine ring are desirable, and the halogen atom therein is desirably a chlorine atom or bromine atom.

### (Synthetic reaction of pyridinepolysulfide)

The polysulfide used may include those obtained beforehand by reacting 1 to 7 moles, desirably 1 to 2 moles of sulfur with one mole of a mixture of an alkali metal hydroxide and a hydrosulfide thereof, or with one mole of an alkali metal sulfide according to the conventional process, and may also include those formed in the reaction system by reacting the above reactants in the presence of the halogeno-substituted pyridine compound of the general formula (II) to be directly used in situ. The alkali metal used in the alkali metal hydroxide, the hydrosulfide thereof or the sulfide thereof may include lithium, sodium and potassium, preferably sodium. The amount to be used of the hydroxide, hydrosulfide or sulfide is 0.75 to 5 moles, desirably 1 to 1.5 moles respectively per one mole of the halogeno-substituted pyridine compound. Normally water may be used as a solvent in the above reaction, but an organic solvent miscible with polysulfide and water may also be used. Examples of the organic solvent may include lower alcohol such as methanol, ethanol and propanol, polyalcohol such as ethylene glycol or propylene glycol, ethers such as tetrahydrofuran, an aprotic polar solvent such as dioxane or dimethylsulfoxide, ketones such as methyl ethyl ketone, nitriles such as acetonitrile, and the like. The amount of the solvent used is normally 10 to 1000% by weight, desirably 10 to 100% by weight based on the halogeno-substituted pyridine compound. Other reaction conditions of the above reaction may not be generally defined, but the reaction temperature is normally 0°C to reflux temperature, desirably 80 to 150°C, the reaction pressure is atmospheric pressure to several atoms, and the reaction time is generally 0.5 to 30 hours.

### (Acid treatment)

Since according to the above reaction, the mercapto-substituted pyridine compound is usually formed as an alkali metal salt of polysulfide, application of the conventional acid treatment to the reaction product results in liberating the intended mercapto-substituted pyridine compound, generating hydrogen sulfide gas, and in forming sulfur. The acid treatment is carried out, for example, by adding a non-oxidative mineral acid such as a concentrated hydrochloric acid or a dilute sulfuric acid to the reaction product in such an amount that the pH therein becomes 3 or lower, followed by subjecting to the conventional purification and separation procedure, resulting in making it possible to isolate the intended mercapto-substituted pyridine compound.

Typical examples of the mercapto-substituted pyridine compound having the general formula (B) and salts thereof are shown as follows:
- Compound No.1:: N,N-dimethyl-2-mercaptonicotinamide, m.p. 214 - 215°C
- Compound No.2:: sodium salt of N,N-dimethyl-2-mercapto-nicotinamide, m.p. 267 - 271°C
- Compound No.3:: N,N-diethyl-2-mercapto-nicotinamide, m.p. 207 - 210°C
- Compound No.4:: sodium salt of N,N-diethyl-2-mercapto-nicotinamide
- Compound No.5:: 4-(N,N-dimethylaminocarbonyl)-2-mercapto-pyridine

Subjecting the above mercapto-substituted pyridine compound or salt thereof to oxidation and halogenation reaction results in obtaining a halogenosulfonyl-substituted pyridine compound of formula (A).

The oxidation and halogenation process may include a process in which the mercapto-substituted pyridine compound or salt, thereof is reacted with a halogenating agent such as chlorine gas, bromine or the like in the presence of water, an aqueous hydrochloric acid solution, acetic acid or an aqueous acetic acid solution; a process in which the mercapto-substituted pyridine compound or salt thereof is reacted with hypochlorite or hypobromite in the presence of water or an aqueous hydrochloric acid solution, and the like, the former being desirable.

In the above processes, an aprotic organic solvent may be used to make simple the post treatment of the reaction. Examples of the solvent used include benzene, hexane, toluene, xylene, methylene chloride, chloroform, carbon tetrachloride, ethylene dichloride, trichloroethylene, diethyl ether, ethyl acetate and the like. The amount of the solvent used is normally 50 to 2000% by weight based on the halogeno-substituted pyridine compound.

In the above processes, the amount of chlorine, bromine, hypochlorite, hypobromite and the like is in the range of from a theoretical amount for reaction to such an amount as to slightly exceed the theoretical amount based on the mercapto-substituted pyridine compound or salt thereof. Similarly thereto, the amount of water, the aqueous hydrochloric acid solution, acetic acid, the aqueous acetic acid solution or the like is 50 to 2000% by weight. The aqueous hydrochloric acid solution or the aqueous acetic acid solution may be used at a concentration of 1 to 30% by weight. In the above processes, other reaction conditions are not generally defined, but the reaction temperature is normally -20 to +50°C, desirably -10 to +10°C, and the reaction time is 0.1 to 5 hours. Application of the conventional purification and separation procedures to the reaction product results in making it possible to separate the halogenosulfonyl-substituted pyridine compound.

Typical examples of the halogenosulfonyl-substituted pyridine compound are shown as follows.
- Compound No.A:: 2-chlorosulfonyl-N,N-dimethylnicotinamide, m.p. 114 - 117°C
- Compound No.B:: 2-bromosulfonyl-N,N-dimethylnicotinamide, m.p. 108 - 111°C
- Compound No.C:: 2-chlorosulfonyl-N,N-diethylnicotinamide, oily substance
- Compound No.D:: 2-bromosulfonyl-N,N-diethylnicotinamide
- Compound No.E:: 2-chlorosulfonyl-N,N-dimethylnicotinamide-1-oxide, m.p. 96.5 - 100°C

Reaction of the halogenosulfonyl-substituted pyridine compound with ammonia gas or ammonia water at -10 to +100°C leads to an aminosulfonyl-substituted pyridine compound, a further reaction of which with 2-isocyanate-4,6-dimethoxypyrimidine or 2-chlorocarbonylamino-4,6-dimethoxy-pyrimidine at -10 to +100°C easily leads to N-[(4,6-dimethoxypyrimidin-2-yl) aminocarbonyl]-3-aminocarboxyl-2-pyridine sulfonamide compound. Application of the pyridine sulfonamide compound in an amount of 0.1 to 100 g per one are makes it possible to effectively control various kinds of harmful weeds and it has such high safety for corn as to be useful as a herbicide for the corn field.

Explanations are given on examples of the present invention as well as preparation examples of pyridine sulfonamide compound.

### Example 1

### (Synthetic reaction of pyridinepolysulfide)

A mixture of 25.0 g (0.1 mole) of 96% sodium sulfide nonahydrate, 10.4 g (0.325 mole) of sulfur and 56 mℓ of water is refluxed by heating. At the time when sulfur dissolves completely to form a homogeneous solution, 18.45 g (0.1 mole) of 2-chloro-N,N-dimethylnicotinamide is added, followed by refluxing by heating for 18 hours to form sodium salt of N,N-dimethyl-nicotinamide-2-polysulfide.

### (Acid treatment)

The above reaction product is allowed to cool down to room temperature, 15 mℓ of concentrated hydrochloric acid is carefully dropped (accompanying generation of hydrogen sulfide and precipitation of sulfur), and after the completion of dropping, agitation is carried out for 15 minutes. Sulfur is filtered off and washed with warm water, and the filtrate and washing liquor are confirmed to be evaporated to dryness under vacuum. The residue is repeatedly extracted with chloroform and dried over anhydrous solution sulfate, and chloroform is distilled off and the residual substance is purified with a silica gel column chromatography (developing solvent: methanol/chloroform = 1/9) to obtain 17.2 g of a yellow crystalline N,N-dimethyl-2-mercaptonicotinamide (m.p. 214-215°C).

### Example 2

### (Synthetic reaction of pyridinepolysulfide)

A mixture of 5.25 g (0.021 mole) of 96% sodium sulfide nonahydrate, 2.2 g (0.069 mole) of sulfur and 5 mℓ of water is refluxed by heating. At the time when the sulfur dissolves completely to form a homogeneous solution, 4.46 g (0.021 mole) of 2-chloro-N,N-diethylnicotinamide is added, followed by being refluxed by heating for 10 hours to form a sodium salt of N,N-diethylnicotinamide-2-polysulfide.

### (Acid treatment)

After the above reaction product is allowed to cool down to room temperature, 4 mℓ of concentrated hydrochloric acid is carefully dropped (accompanying generation of hydrogen sulfide and precipitation of sulfur). After the completion of dropping procedure, agitation is further carried out for 15 minutes. The aqueous phase is extracted with dichloromethane, followed by dry and distilling off the solvent under vacuum and purification of the residue with a silica gel column chromatography (developing solvent: methanol/chloroform = 1/19) to obtain 4.09 g of an yellow crystalline N,N-diethyl-2-mercaptonicotinamide (m.p. 207-210°C).

### Example 3

### (Synthetic reaction of pyridinepolysulfide)

A mixture of 24 g (0.3 mole) of sodium hydrosulfide of 70% purity, 9.6 g (0.3 mole) of sulfur, 12 g (0.3 mole) of sodium hydroxide and 15 mℓ of water is refluxed by heating. At the time when the sulfur dissolves completely to form a homogeneous solution, 55.4 g (0.3 mole) of 2-chloro-N,N-dimethylnicotinamide is added, followed by being refluxed (125°C) by heating for 2 hours to form a sodium salt of N,N-dimethylnicotinamide-2-polysulfide.

### (Acid treatment)

After the above reaction product is allowed to cool down to room temperature, 150 mℓ of water is added and about 30 mℓ of concentrated hydrochloric acid is dropped so as to adjust to pH 2, while hydrogen sulfide generates and sulfur precipitates. After the completion of dropping, agitation is carried out at 60 to 70°C for 30 minutes, followed by filtering off the sulfur while warming and by washing the residue with 150 mℓ of warm water to obtain filtrate and washing liquor which contain N,N-dimethyl-2-mercapto-nicotinamide.

### (Oxidation and bromination reaction)

The above mixture of filtrate and washing liquor are cooled with a mixture of sodium chloride and ice, 147 g (0.92 mole) of bromine is dropped with stirring at 5°C or lower, 100 mℓ of a cold water is then charged to be stirred, at -5°C for one hour. The reaction product is extracted with 700 mℓ of cold methylene chloride to obtain an extract liquor containing 2-bromosulfonyl-N,N-dimethylnicotinamide.

### Example 4

The oxidation and bromination reaction in Example 3 is varied as follows. Into 300 mℓ of water is suspended 54.6 g (0.3 mole) of N,N-dimethyl-2-mercaptonicotinamide to be cooled with agitation, and 144 g (0.9 mole) of bromine is dropped at -6°C to 0°C. After the completion of dropping above, purification procedure is carried out in the same manner as in Example 3 to obtain a methylene chloride solution containing 2-bromosulfonyl-N,N-dimethylnicotinamide. The methylene chloride solution is washed with 300 mℓ of ice water and 200 mℓ of cooled 1% aqueous solution of sodium thiosulfate, methylene chloride is distilled off at an inner temperature of 30°C or lower, followed by drying under vacuum to obtain 76 g of reaction product. The reaction product is dissolved in warm ethylene dichloride and is recrystallized with n-hexane to obtain 64 g (yield: 72.8%) of 2-bromosulfonyl-N,N-dimethylnicotinamide (m.p. 108-111°C).

### Example 5

### (Synthetic reaction of pyridinepolysulfide)

A mixture of 55.4 g of 2-chloro-N,N-dimethylnicotinamide, 24 g of sodium hydrosulfide of 70% purity, 9.6 g of sulfur, 12 g of sodium hydroxide and 15 mℓ of water is refluxed by heating with agitation for about 2 hours to form a sodium salt of N,N-dimethylnicotinamide-2-polysulfide.

### (Acid treatment)

To the above reaction product are added 150 mℓ of water and 30 mℓ of a 50% aqueous sulfuric acid solution to be stirred at 60 to 70°C for 30 minutes, and the precipitated sulfur is filtered off with warming. The sulfur is washed with 100 mℓ of warm water, and the filtrate and washing liquor are combined to obtain a solution containing N,N-dimethyl-2-mercaptonicotinamide.

### (Oxidation and bromination reaction)

The above solution is cooled down to 0°C or lower, 350 mℓ of methylene chloride is added, 144 g of bromine is dropped over about 20 minutes, and a methylene chloride layer is separated to obtain a methylene chloride solution of 2-bromosulfonyl-N,N-dimethylnicotinamide.

### Example 6

### (Synthetic reaction of pyridinepolysulfide)

A mixture of 26.4g (0.105 mole) of 96% sodium sulfide nonahydrate, 10.8g (0.338 mole) of sulfur and 56 mℓ of water is refluxed by heating. At the time when a homogeneous solution is formed, 18.45g (0.1 mole) of 2-chloro-N,N-dimethylnicotinamide is added, followed by refluxing by heating for 20 hours to form a sodium salt of N,N-dimethyl-nicotinamide-2-polysulfide.

### (Acid treatment)

The above reaction product is allowed to cool down to room temperature, 15 mℓ of concentrated hydrochloric acid is then carefully dropped. After the completion of dropping, stirring is carried out for 15 minutes to form N,N-dimethyl-2-mercaptonicotinamide.

### (Oxidation and chlorination reaction)

The resulting insoluble matter is filtered off and washed with warm water, the filtrate and washing liquor are combined, about 50 mℓ of dichloromethane is added to the combined filtrate and washing liquor to be ice-cooled, and chlorine gas is introduced thereinto. After confirming disappearance of N,N-dimethyl-2-mercaptonicotinamide, introduction of chlorine gas is stopped to form 2-chlorosulfonyl-N,N-dimethylnicotinamide (m.p. 114-117°C). The reaction mixture is charged into ice water, the dichloromethane layer is separated and collected, the collected dichloromethane layer is combined with one obtained by extracting the water layer with dichloromethane to be washed with water, followed by drying over anhydrous sodium sulfate and by cooling with ice water again.

### (Amidation reaction)

Ammonia gas is introduced into the resulting dichloromethane layer at 10°C or lower. At the time when the reaction mixture becomes weakly alkaline, introduction of ammonia gas is stopped. Dichloromethane is distilled off the reaction product, the remaining white crystals are washed with ethyl acetate followed by water, and dried to obtain 15.5 g of 2-aminosulfonyl-N,N-dimethylnicotinamide (m.p. 209-211°C).

### (Condensation)

A mixed solution containing 250 mg of 2-amino-4,6-dimethoxypyrimidine, 0.65 g of triethylamine and 2.5 g of ethyl acetate is dropped at 15°C into 6.3 g of an ethyl acetate solution of 20% phosgene to be reacted for one hour keeping the temperature at 15°C, followed by warming in an oil bath at 90°C to distill off excessive phosgene and ethyl acetate, adding a solution prepared by dissolving 300 mg of 2-aminosulfonyl-N,N-dimethylnicotinamide in 10 mℓ of acetonitrile, and by dropping 0.2 g of triethylamine to be reacted for one hour at room temperature.

After the completion of the reaction, the reaction product is charged into water, followed by acidifying with hydrochloric acid, filtering off deposited crystals, washing with water, and by drying to obtain 0.46 g of N-[(4,6-dimethoxypyrimidine-2-yl) aminocarbonyl]-3-dimethyl-aminocarbonyl-2-pyridinesulfonamide (m.p. 169-173°C).

### Example 7

A field soil is packed in a 1/1,500 are pot, and seeds of various kinds of plants are sowed thereon. When respective plants reach certain plant stages in leaf number respectively (corn : 3.2 leaf stage, wheat : 3.5 leaf stage, common cocklebur : 2.5 leaf stage, tall morningglory : 1.0 leaf stage, smartweed : 1.2 leaf stage, prickly sida : 1.0 leaf stage, slender amaranth : 0.5 leaf stage, barnyard grass: 2.0 leaf stage), a wettable powder of N-[(4,6-dimethoxypyrimidine-2-yl)aminocarbonyl]-3-dimethylaminocarbonyl-2-pyridinesulfonamide is weighed by such an amount as to be 1.25 (g/a) (as an amount of the active ingredient) to be diluted with 5L of water per one are, followed by adding to the aqueous solution, an agricultural spreader by such an amount as to be 0.2% to be subjected to foliar application with a small-sized sprayer. Twenty four days after application, growth conditions of respective plants are observed visually and a degree of growth control is evaluated by ten grades (1 : the same as in non-application area to 10 : complete control). The results are shown below.

| Plants | Degree of Growth Control |
|---|---|
| Corn | 1 |
| Wheat | 8 |
| Common cocklebur | 10 |
| Tall morningglory | 8 |
| Prickly sida | 7 |
| Smartweed | 8 |
| Slender amaranth | 10 |
| Barnyard grass | 10 |

## Claims

1. A process for producing a compound of the general formula (A): 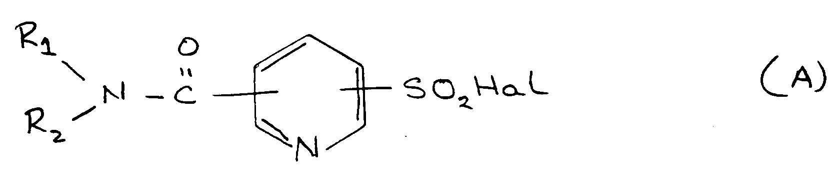 wherein each of R₁ and R₂ is a C₁-C₆ alkyl group, and Hal is chlorine or bromine;
said process comprising oxidising and halogenating a compound of the general formula (B): 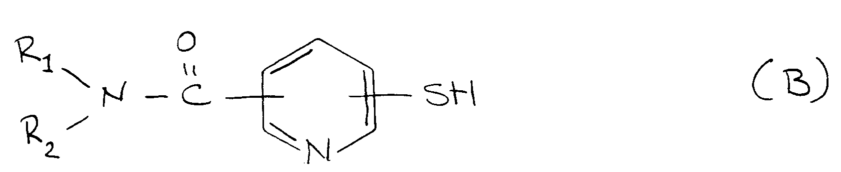 wherein R₁ and R₂ are as defined above, or a salt thereof.

2. A process as claimed in claim 1, wherein the compound of the formula (A) is a compound of the formula (A') 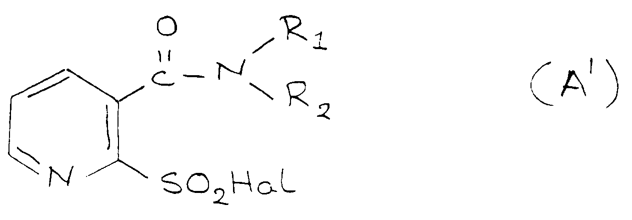 and the compound of the formula (B) is a compound of the formula (B')

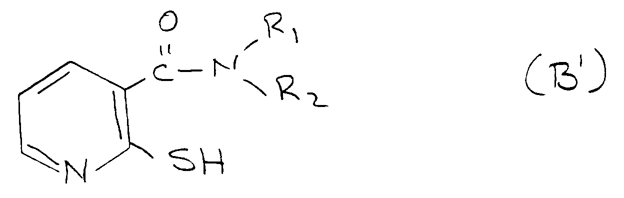

3. A process as claimed in claim 1, wherein the compound (A) is 2-chlorosulfonyl-N,N-dimethyinicotinamide.

4. A process as claimed in claim 1, wherein the compound (A) is 2-bromosulfonyl-N,N-dimethylnicotinamide.

## Patentansprüche

1. Verfahren zur Herstellung einer Verbindung der allgemeinen Formel (A) oder eines ihrer Salze: 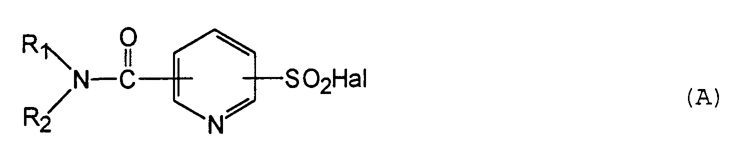 worin R₁ und R₂ jeweils eine C₁-C₆-Alkylgruppe sind und Hal Chlor oder Brom ist;
umfassend die Oxidation und Halogenierung einer Verbindung der allgemeinen Formel (B): 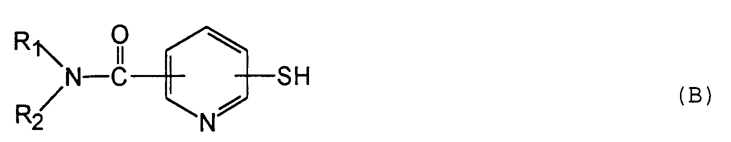 worin R₁ und R₂ wie oben definiert sind.

2. Verfahren gemäß Anspruch 1, worin die Verbindung der Formel (A) eine Verbindung der Formel (A') ist: 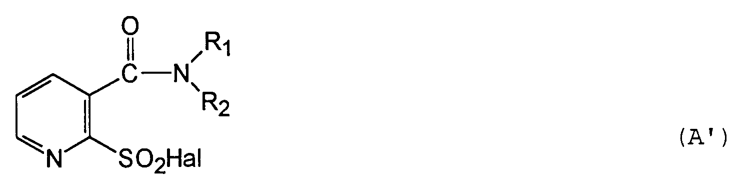 und die Verbindung der Formel (B) eine Verbindung der Formel (B') ist:

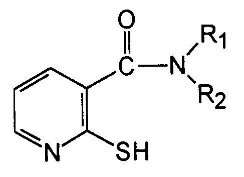

3. Verfahren gemäß Anspruch 1, worin die Verbindung (A) 2-Chlorsulfonyl-N,N-Dimethylnicotinamid ist.

4. Verfahren gemäß Anspruch 1, worin die Verbindung (A) 2-Bromsulfonyl-N,N-Dimethylnicotinamid ist.

## Revendications

1. Procédé pour produire un composé de formule générale (A) 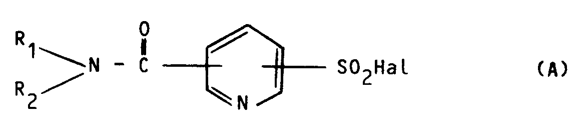 dans laquelle R₁ et R₂ sont chacun un groupe alkyle en C₁-C₆ et Hal est le chlore ou le brome ;
ledit procédé comprenant l'oxydation et l'halogénation d'un composé de formule générale (B) : 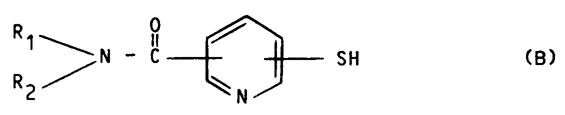 dans laquelle R₁ et R₂ sont définis comme ci-dessus, ou un de ses sels.

2. Procédé selon la revendication 1, dans lequel le composé de formule (A) est un composé de formule (A') : 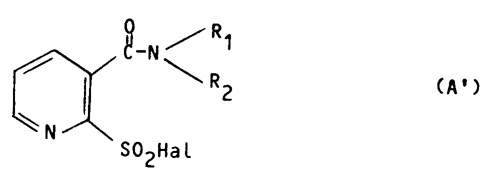 et le composé de formule (B) est un composé de formule (B')

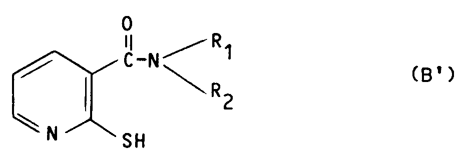

3. Procédé selon la revendication 1, dans lequel le composé (A) est le 2-chlorosulfonyl-N,N-diméthylnicotinamide.

4. Procédé selon la revendication 1, dans lequel le composé (A) est le 2-bromosulfonyl-N,N-diméthylnicotinamide.
